Europäisches Patentamt

European Patent Office

Office européen des brevets

(11) Publication number: **0 153 523**
**B1**

(12) **EUROPEAN PATENT SPECIFICATION**

(45) Date of publication of patent specification: **09.11.88**

(51) Int. Cl.⁴: **A 61 F 2/34**

(21) Application number: **84304092.4**

(22) Date of filing: **18.06.84**

(54) Acetabular cup.

(30) Priority: **14.02.84 GB 8403824**

(43) Date of publication of application:
**04.09.85 Bulletin 85/36**

(45) Publication of the grant of the patent:
**09.11.88 Bulletin 88/45**

(84) Designated Contracting States:
**AT BE CH DE FR GB IT LI LU NL SE**

(56) References cited:
**DE-A-2 911 754**
**DE-A-3 215 583**
**GB-A-1 460 813**
**US-A-3 584 318**

(73) Proprietor: **HOWMEDICA INTERNATIONAL INC.**
**Shannon Industrial Estate**
**Shannon Co. Clare (IE)**

(72) Inventor: **Buning, Onno Jurjen**
**Ferrybridge**
**Clarina Co. Limerick (IE)**

(74) Representative: **Bridge-Butler, Alan James**
**G.F. REDFERN & CO. High Holborn House 52/54**
**High Holborn**
**London WC1V 6RL (GB)**

Courier Press, Leamington Spa, England.

## Description

This invention relates to a reinforced acetabular cup for use with a hip prosthesis and which is held in place in the acetabular socket by cement.

Acetabular cups fall into two broad categories, those that are intended to be held in place by nails or other mechanical devices which engage the bone, and acetabular cups which are held in place by bone cement. The particular type of cup which is to be used for any prosthetic implant is usually selected by the surgeon depending upon many, and variable, factors, for example, it is obviously difficult to use a cup of the first type if the bone is fragile and in this case a cemented cup is usually employed. The techniques which are used for securing an acetabular cup in place will depend upon the manufacturer of the cup and the instructions which are usually followed meticulously by the surgeon concerned. The techniques of application can be complicated and for this reason detailed instructions are always supplied by the manufacturer.

United States Patent Specication 3 584 318 shows a cup construction in which an inner and outer cup are both made from metal and the outer cup is provided with means for attachment by means of screws or nails, fixing holes being provided in the outer cup to allow the nails to pass through them, and, German Patent Specification 2 911 754 also shows a cup construction with a metallic outer shell but again in this construction it is intended to hold the cup in place by concentric ribs which can engage the bone without the use of cement. In fact, this construction is specifically referred to as being cement free. Both these constructions are therefor of the kind which do not employ cement and which require a different technique to implant. In German Patent 2 911 754 openings are shown in the inner liner which are aligned with openings in the outer liner to allow tissue fluid to discharge from the joint and into the cup itself.

It will be appreciated that none of the constructions shown in German Patent 2 911 754 or US Patent 3 584 318 are suitable for implantation with cement.

It is known to use a cup made from a synthetic plastics material and to hold it in place by cement. In certain circumstances these cups have been successful but in others they are not stiff enough and in order to provide the necessary stiffening cups of this type have been known to be provided with an outer shell made of metal, for example cobalt chrome alloy or titanium. Thus the cup comprises an outer shell of metal and an inner liner made from a synthetic plastics material, for example ultra-high molecular weigth polyethylene (UHMWPE). Cups of this type have been used for both types of implantation technique referred to above.

British Patent 1 460 813 shows a cement implantation type cup made from a synthetic plastics material and which is used with a bearing support which is embedded in a surgically prepared cavity in the pelvis. This bearing support comprises an element having a hollow domed part which defines a recess and which can receive the artificial synthetic plastics socket. The bearing support has an outwardly extending flange which lies adjacent and extends around the mouth of the recess for bearing against the bone surrounding the prepared cavity. Thus, the intention of this construction is to provide what is, in effect, a reinforecment for the cavity itself and so that the loading is transferred to the rim of the cavity. The hip joint socket is first prepared, the bearing support is cemented in place and the synthetic plastics material socket is then placed in the bearing support in the required angular position. The bearing support can be provided with at least one aperture and the specification states that the external surface of the socket can be provided with different indentations so that it can be inserted with bone cement. This specification therefore discloses means for reinforcing the cavity in the pelvis and for subsequently placing in the reinforced cavity a synthetic plastics socket. Such a synthetic platics socket may however suffer from insufficient stiffness even in the reinforced opening and the cup may itself be of generally know kind.

The present invention is intended to provide a pre-assembled reinforced acetabular cup of the kind for location by cement and comprising a shell and a liner of improved construction which will not distort as excessively as the more conventional UHMWPE cups. It is therefore aimed to combat acetabuar cup loosening and an additional advantage of the present invention is that a keying feature is provided which will secure the cup in its cement bedding when in use.

According to the present invention a reinforced acetabular cup for use with a hip prosthesis and for anchorage by cement in a prepared cavity in the pelvis comprises a liner made from a synthetic plastics material and having an inner bearing surface and an outer surface provided with blind keying cavities which extend into the liner for a distance less than its thickness, and a reinforcing outer shell having opening therein which is made from a bio-compatible material which is stiffer than the material of the liner and means for securing the shell to the liner with the cavities in the liner aligned with the openings and each cavity being dimensioned to extend beneath the whole area of its co-operating opening in the shell with which is is aligned.

When the cup is cemented in place the cement will pass through the openings in the shell and fill the cavities in the liner and will key the shell to the cement bed.

The openings in the shell may be of any convenient form, for example, merely circular holes, but they are preferably in the form of slots.

With this type of construction the blind keying cavities in the liner can be in the form of grooves and/or sockets.

In one convenient construction the blind keying cavities in the liner are under cut to thus provide a better keying.

If desired the dimensions of at least one of the blind keying cavities in a direction across the mouth thereof is greater than the dimension of the co-operating opening in the shell in the same direction so that at least part of the edge of each opening provides a flange protruding over the blind keying cavity.

Preferably the liner has a location spigot adapted to enter a location opening in the shell and which is riveted over when assembled thereto. The shell can be provided with protrusions to act as cement spacers in an acetabular socket when in use.

Means can be provided to prevent relative rotation of the shell and liner.

The shell can be made from any convenient bio-compatible material and in a convenient construction it is made from a metal, for example Vitallium (Registered Trade Mark of Howmedica Inc.).

Alternatively the shell could be made from carbon fibre or carbon fibre impregnated plastics material.

Preferably the shell is thinner than 1.5 mm and can be 1.1 mm.

The liner can be made from any convenient synthetic plastics material and in a preferred construction it is made from ultra-high molecular weight polyethylene (UHMWPE).

The invention also includes a total hip prosthesis comprising an acetabular cup as set forth in combination with a prosthetic femoral head device.

The invention can be performed in many ways and two embodiments will now be described by way of example and with reference to the accompanying drawings in which:

Figure 1 is a side elevation of an acetabular cup according to the invention with a prosthetic femoral head device in place;

Figure 2 is a plan view of the cup shown in Figure 1;

Figure 3 is a part cross-sectional view of the construction shown in Figures 1 and 2;

Figure 4 is a part cross-sectional view of an alternative key cavity construction;

Figure 5 is a cross-sectional view of the liner before assembly; and,

Figure 6 is a cross-sectional view of the shell before assembly.

As shown in the drawings the acetabular cup according to the invention comprises a cup-shaped liner 1 which has an inner part-spherical socket 2. The mouth of the socket 2 is enlarged as shown at 3 and the liner has an outer flange 4. Immediately above the flange 4 is a groove 5 the purpose of which will be described later. The outer surface of the liner is provided with three grooves 6, 7 and 8 which act as blind keying cavities and location spigot 9 is provided at the head of the liner.

It will be seen that the general outer shape of the liner is part-spherical and it is made from any suitable synthetic plastics material, for example, UHMWPE.

The liner 1 is adapted to be received in a reinforcing outer shell 10 made from a suitable bio-compatible material, for example Vitallium (Registered Trade Mark of Howmedica Inc.). The thickness of the metal is less than 1.5 mm and can, for example, be 1.1 mm.

The shell 10 is provided with a series of substantially parallel interrupted slots 11, 12 and 13 which are aligned with the blind keying cavities 6, 7 and 8 when the liner is assembled within the shell.

The inner surface of the shell is shaped to closely receive the liner 1 and is provided with an opening 14 to receive the location spigot 9. The outer surface of the shell is provided with four knob-like protrusions 15 to assist in maintaining an even wall thickness of cement around the cup when in location in a prepared acetabular socket when in use.

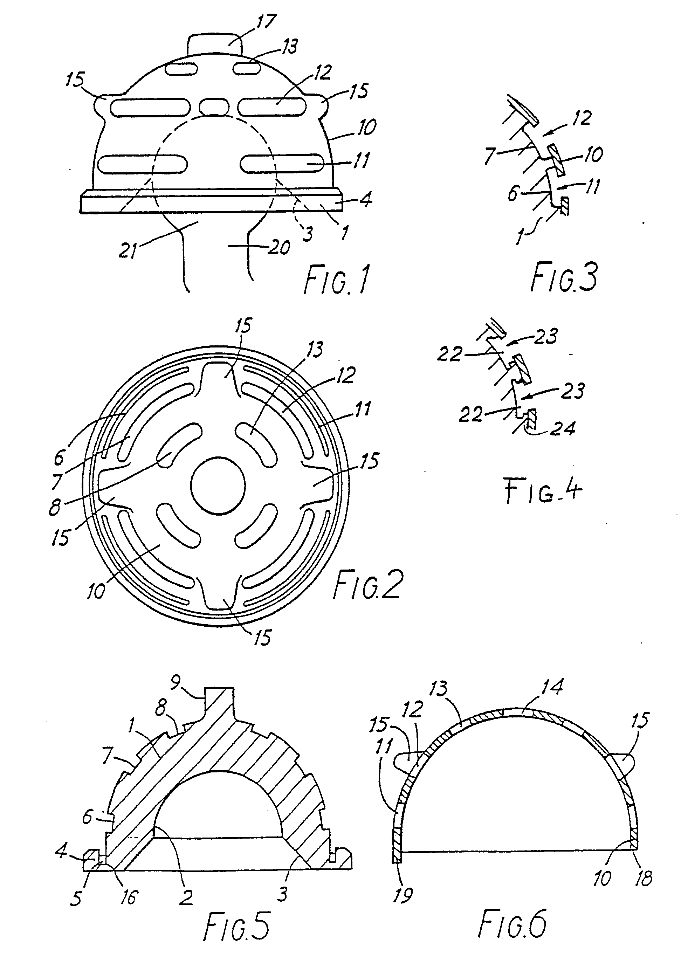

As is most clearly shown in Figure 3 the dimensions of the blind keying cavites in a direction across the mouth thereof is greater than the dimensions of the co-operating openings 11, 12, 13 in the shell 10.

In order to fasten the liner to the shell the spigot 9 is rivetted over, as shown at 17 in Figure 1, and in order to prevent relative rotation of the parts the rim 18 of the shell which is located in the groove 5 is provided with a lug 19 which engages a socket 16 in the groove. In an alternative arrangement (not shown) a longitudinal key on the inside of the shell co-operates with a longitudinal groove in the liner.

In Figure 1 a prosthetic femoral head device 20 is shown in place in the cup, the ball 21 of the femoral head device being located in the socket 2. When an acetabular cup according to the invention is to implanted the acetabular socket of the patient is first prepared and the acetabular socket is lined with suitable cement. The cup is pushed into place and the cement passes through openings 11, 12 and 13 in the shell and fills the blind keying cavities 6, 7 and 8, thus keying the shell to the cement bed.

Figure 4 shows an alternative construction for the blind keying cavities which are indicated by reference numeral 22, the shell 10 being provided with suitable openings, indicated by reference numeral 23. It will be seen that these blind keying cavities are undercut to provide flanges 24 adjacent their mouths. This type of cavity provides an even better keying effect, the cement keying the liner to the shell and to the cement bed.

If desired the grooves, 6, 7 and 8 could be replaced by blind sockets formed for example as interrupted grooves and which are aligned with the interrupted slots 11, 12 and 13 so as to act as keying cavities, or a combination of grooves and sockets could be used.

An alternative material for manufacture of the outer shell could be carbon fibre or carbon fibre reinforced plastics material which could provide the necessary stiffness.

## Claims

1. A reinforced acetabular cup for use with a hip prosthesis and for anchorage by cement in a prepared cavity in the pelvis comprising a liner (1) made from synthetic plastics material and having an inner bearing surface and an outer surface provided with blind keying cavities (6—8; 22) which extend into the liner (1) for a distance less than its thickness, and a reinforcing outer shell (10) havings openings (11—13; 23) therein which is made from a bio-compatible material which is stiffer than the material of the liner (1) and having means (9) (14) for securing the shell (10) to the liner (1) with the cavities (6—8; 22) in the liner aligned with the openings (11—13; 23) and each cavity (6—8; 22) being dimensioned to extend beneath the whole area of its co-operating opening (11—13; 23) in the shell with which it is aligned.

2. An acetabular cup as claimed in claim 1 in which the openings (11—13; 23) in said shell (10) are in the form of slots.

3. An acetabular cup as claimed in claim 2 in which the blind keying cavities (6—8; 23) in said liner are in the form of grooves and/or sockets.

4. An acetabular cup as claimed in claims 1 to 3 in which the blind keying cavities (22) in said liner (1) are undercut.

5. An acetabular cup as claimed in any one of the preceding claims in which the dimensions of at least one of the blind keying cavities (6—8) in a direction across the mouth thereof is greter than the dimensions of the co-operating opening (11—13) in the shell (10) in the same direction so that at least part of the edge of each opening (11—13) provides a flange protruding over the blind keying cavity.

6. An acetabular cup as claimed in any one of the preceding claims 1—5 in which the liner (1) has a location spigot (9) adapted to enter a location opening (14) in the shell (10) and which is rivetted over to secure it thereto.

7. An acetabular cup as claimed in claims 1 to 6 in which said shell (10) is provided with protrusions (15) to act as cement spacers in an acetabular socket.

8. An acetabular cup as claimed in Claims 1 to 7 having means (16, 19) to prevent relative rotation of the shell (10) and liner (1).

9. An acetabular cup as claimed in any one of the preceding claims in which the shell (10) is made from a metal carbon fibre, or carbon fibre impregnated plastics material.

10. An acetabular cup as claimed in any one of the preceding claims in which the shell (10) is thinner than 1.5 mm.

11. An acetabular cup as claimed in claim 11 in which the shell (10) is 1.1 mm thick.

12. An acetabular cup as claimed in any one of the preceding claims in which the liner (1) is made from ultra-high molecular weight polyethylene UHMWPE.

13. A total hip prosthesis having an acetabular cup as claimed in any one of the preceding claims in combination with a prosthetic femoral head device.

## Patentansprüche

1. Verstärkte Acetabulum-Pfanne zur Verwendung mit einer Hüftprothese und zur Verankerung mit Zement in einem vorbereiteten Hohlraum im Becken, umfassend eine Buchse (1) aus synthetischem Kunststoff mit einer inneren Lagerfläche und einer äusseren Oberfläche mit blinden Verschlusshohlräumen (6—8; 22), die sich in die Buchse (1) erstrecken in eine Tiefe, die geringer ist als deren Dicke, eine verstärkende äussere Schale (10) mit Oeffnungen (11—13; 23), bestehend aus einem bioverträglichen Material, das steifer ist als das Material der Buchse (1) sowie Mittel (9) (14) zur Befestigung der Schale (10) an der Buchse (1), wobei die blinden Verschlusshohlräume (6—8; 22) so angeordnet sind, dass sie mit den Oeffnungen (11—13; 23) ausgerichtet sind und jeder blinder Verschlusshohlraum (6—8; 22) so bemessen ist, dass er sich unterhalb der gesamten Fläche der mit ihm zusammenwirkenden Oeffnung (11—13; 23) in der Schale, mit der er ausgerichtet ist, erstreckt.

2. Acetabulum-Pfanne nach Anspruch 1, bei der die Oeffnungen (11—13; 23) in besagter Schale (10) die Form von Schlitzen aufweisen.

3. Acetabulum-Pfanne nach Anspruch 2, bei der die blinden Verschlusshohlräume (6—8; 22) in besagter Buchse die Form von Rillen und/oder Schlitzen aufweisen.

4. Acetabulum-Pfanne nach den Ansprüchen 1 bis 3, bei der die blinden Verschlusshohlräume (22) in der Buchse (1) unterschnitten sind.

5. Acetabulum-Pfanne nach einem der vorhergehenden Ansprüche, bei der mindestens einer der blinden Verschlusshohlräume (6—8) quer zu seiner Mündung grösser ist als die mit ihm zusammenwirkende Oeffnung (11—13) in der Schale (10), in der gleichen Richtung, so dass wenigstens ein Teil des Randes jeder Oeffnung (11—13) einen Flansch bildet, der über den blinden Verschlusshohlraum hinausragt.

6. Acetabulum-Pfanne nach einem der vorhergehenden Ansprüche 1 bis 5, bei der die Buchse (1) über einen Befestigungszapfen (9) verfügt, der in eine Aufnahmeöffnung (14) in der Schale (10) passt und mit ihr zum Zweck der Verbindung vernietet wird.

7. Acetabulum-Pfanne nach den Ansprüchen 1 bis 6, bei der besagte Schale (10) mit Ausbuchtungen (15) versehen ist, die in einer Acetabulum-Vertiefung als Zementdistanzhalter wirken.

8. Acetabulum-Pfanne nach den Ansprüchen 1 bis 7, die über Mittel (16, 19) verfügt, die eine relative Rotation zwischen der Schale (10) und der Buchse (1) verhindern.

9. Acetabulum-Pfanne nach einem der vorhergehenden Ansprüche, bei der die Schale (10) aus einem Metall, aus Kohlefaser oder aus einem mit Kohlefaser verstärkten Kunststoff besteht.

10. Acetabulum-Pfanne nach einem der vorhergehenden Ansprüche, bei der die Schale (10) dünner als 1.5 mm ist.

11. Acetabulum-Pfanne nach Anspruch 11, bei der die Schale (10) 1.1 mm dick ist.

12. Acetabulum-Pfanne nach einem der vorhergehenden Anspüche, deren Buchse (1) au Polyaethylen mit extrem hohem Molekulargewicht (UHMWPE) besteht.

13. Hüftprothese, umfassend eine Acetabulum-Pfanne nach einem der vorhergehenden Ansprüche kombiniert mit einer Oberschenkelgelenkkopfprothese.

**Revendications**

1. Coque acétabulaire renforcée pour utilisation avec une prothèse de la hanche et pour ancrage par du ciment dans une cavité préparée dans le bassin comprenant un revêtement (1) fait d'une matière plastique synthétique et ayant und surface d'appui interne et une surface externe munie de cavités de calage aveugles (6—8; 22) qui s'étendent dans le revêtement (1) sur une distance inférieurè à son epaisseur, et une enveloppe externe de renforcement (10) comportant des ouvertures (11—13; 23) qui est faite d'une matière bio-compatible qui est plus rigide qui la matière du revêtement (1) et ayant des moyens (9) (14) pour fixer l'enveloppe (10) au revêtement (1), les cavités (6—8; 22) du revêtement étant alignées avec les ouvertures (11—13; 23) et chaque cavité (6—8, 22) étant dimensionnée pour s'étendre en-dessous de toute la surface de l'ouverture qui coopère avec elle (11—13; 23) dans l'enveloppe avec laquelle elle est alignée.

2. Coque acétabulaire selon la revendication 1 dans laquelle les ouvertures (11—13; 23) de ladite enveloppe (10) sont sous forme de fentes.

3. Coque acétabulaire selon la revendication 2 dans laquelle les cavités de calage aveugles (6—8; 22) dudit revêtement sont sous la forme des rainures et/ou de douilles.

4. Coque acétabulaire selon les revendications 1 à 3 dans laquelle les cavités de calage aveugles (22) dans ledit manchon (1) sont sous-cavées.

5. Coque acétabulaire selon l'une quelconque des revendications précédentes dans laquelle la dimension d'au moins une des cavités de calage aveugles (6—8) dans un sens transversal par rapport à leur ouverture est supérieure à la dimension de l'ouverture qui coopère avec elle (11—13) dans l'enveloppe (10) dans le même sens de manière qu'au moins une partie du bord de chaque ouverture (11—13) fournit une collerette faisant saillie au-dessus de la cavité de calage aveugle.

6. Coque acétabulaire selon l'une quelconque des revendications 1—5 précédentes dans laquelle le revêtement (1) a une saillie d'emboîtement (9) adaptée pour pénétrer dans une ouverture de positionnement (14) dans l'enveloppe (10) et que est rivetée de manière à y être fixée.

7. Coque acétabulaire selon les revendications 1 à 6 dans laquelle ladite enveloppe (10) est munie d'avancées (15) pour servir d'entretoises de ciment dans une douille acétabulaire.

8. Coque acétabulaire selon les revendications 1 à 7 ayant des moyens pour empêcher une rotation relative de l'enveloppe (10) et du manchon (1).

9. Coque acétabulaire selon l'une quelconque des revendications précédentes dans laquelle l'enveloppe (10) est faite de métal, d'une fibre de carbone ou d'une matière plastique imprégnée de fibre de carbone.

10. Coque acétabulaire selon l'une quelconque des revendications précédentes dans laquelle l'enveloppe (10) a moins de 1,5 mm d'épaisseur.

11. Coque acétabulaire selon la revendication 11 dans laquelle l'enveloppe (10) a 1,1 mm d'épaisseur.

12. Coque acétabulaire selon l'une quelconque des revendications précédentes dans laquelle le manchon (1) est fait de polyéthylène à très haut poids moléculaire ("ultra-high molecular weight polyethylene"-UHMWPE).

13. Prothèse totale de hanche ayant une coque acétabulaire selon l'une quelconque des revendications précédentes en combinaison avec un appareil prothétique de tête fémorale.

0 153 523

FIG.1

FIG.3

FIG.2

FIG.4

FIG.5

FIG.6